# EUROPEAN PATENT SPECIFICATION

(11) **EP 4 259 119 B1**
(45) Date of publication and mention of the grant of the patent: **06.05.2026**
(21) Application number: 21823935.8
(22) Date of filing: 10.12.2021
(51) Int. Cl.: A61K 31/195, A61K 9/00, A61K 9/107, A61K 33/18, A61K 36/66, A61K 49/00, A61P 19/00, A61P 19/02, A61P 29/00, A61K 31/201, A61K 49/04, A61L 24/00, A61L 24/04

(54) **EMBOLIZING EMULSION FOR TREATMENT OF INFLAMMATORY HYPERVASCULARIZATION ASSOCIATED WITH MUSCULOSKELETAL DISORDERS**
EMBOLISIERENDE EMULSION ZUR BEHANDLUNG ENTZÜNDLICHER HYPERVASKULARISATION IN ZUSAMMENHANG MIT MUSKEL-SKELETT-ERKRANKUNGEN
ÉMULSION EMBOLISANTE POUR LE TRAITEMENT DE L'HYPERVASCULARISATION INFLAMMATOIRE ASSOCIÉE AUX TROUBLES MUSCULO-SQUELETTIQUES

(30) Priority: 10.12.2020 EP 20306538; 26.02.2021 EP 21305228
(43) Date of publication of application: 18.10.2023
(73) Proprietor: Assistance Publique - Hôpitaux de Paris, 75012 Paris (FR); Guerbet, 93420 Villepinte (FR); Université Paris Cité, 75006 Paris (FR)
(72) Inventor: SAPOVAL, Marc, 94110 ARCUEIL (FR); DEL GIUDICE, Constantino, 75015 PARIS (FR); DEAN, Carole, 60700 SAINT-MARTIN-LONGUEAU (FR); PELLERIN, Olivier, 75016 PARIS (FR)
(74) Representative: Regimbeau
(86) International application number: PCT/EP2021/085284
(87) International publication number: WO 2022/123049

(56) References cited:
- WO-A1-2020/231842
- OKUNO YUJI ET AL: "Transcatheter Arterial Embolization Using Imipenem/Cilastatin Sodium for Tendinopathy and Enthesopathy Refractory to Nonsurgical Management", JOURNAL OF VASCULAR AND INTERVENTIONAL RADIOLOGY, ELSEVIER, AMSTERDAM, NL, vol. 24, no. 6, 21 May 2013 (2013-05-21), pages 787 - 792, XP028549667, ISSN: 1051-0443, DOI: 10.1016/J.JVIR.2013.02.033
- HORI S ET AL: "LIPIODOL-IOPAMIDOL EMULSION. NEW CONTRAST MATERIAL FOR CT OF THE LIVER AND SPLEEN IODINAED OIL EMULSIFIED BY A NON-IONIC CONTRAST MATERIAL", NIPPON IGAKU HOSHASEN GAKKAI ZASSHI - NIPPON ACTA RADIOLOGICA, TOKYO, JP, vol. 48, no. 12, 1 January 1988 (1988-01-01), pages 1503 - 1510, XP000906834, ISSN: 0048-0428
- DATABASE BIOSIS [online] BIOSCIENCES INFORMATION SERVICE, PHILADELPHIA, PA, US; May 1999 (1999-05-01), HORIO KEIJI ET AL: "Effect of temporary hepatic venous occlusion on hepatic arterial embolization: An experimental study", XP002802912, Database accession no. PREV199900419243
- INVESTIGATIVE RADIOLOGY, vol. 34, no. 5, May 1999 (1999-05-01), pages 341 - 347, ISSN: 0020-9996

## Description

### Background

The present invention is in the field of musculoskeletal disorders. It more particularly relates to an embolizing composition for use in the treatment of inflammatory hypervascularization associated with musculoskeletal disorders.

Musculoskeletal disorders (MSDs) affect the bones, muscles, joints, tendons, and/or ligaments. They are the most frequent cause of disability worldwide and result in millions of outpatient visits each year (James et al., 2017). Indeed, in the US alone an estimated 50% of adults are affected by MSDs, with MSDs outranking all other conditions as a cause of lost work days (United States Bone and Joint Initiative, 2018). The most common MSDs include arthritis, lower back and neck pain, and trauma (e.g., from repetitive motion, overuse, falls) which results in damage to soft tissues and/or bone fractures. Of particular note, joint disorders, such as tennis elbow, frozen shoulder, and arthritis of the knee or wrist, are a common cause of disability in relatively young, active patients.

Pain is one of the earliest and most common symptoms of MSDs, and is often associated with inflammation of the affected area, along with stiffness, tenderness, weakness, and/or swelling or deformation. In the case of inflammatory joint pathologies, intractable pain is notably associated with a partial or complete loss of function.

First-line treatment of MSDs includes the administration of pharmaceutical compositions (e.g., analgesics, including opioids, nonsteroidal anti-inflammatory drugs, muscle relaxants, corticosteroid injections), which are generally accompanied by non-pharmaceutical treatment methods (e.g., use of hot/cold packs, physical therapy, exercises, self-management advice). However, long-term medication can lead to liver and/or renal dysfunction, ulcer formation, and even addiction in the case of opioids. Furthermore, treatment efficacy varies significantly from one patient to the next, making it difficult to establish a set protocol, and refractory MSD is frequent. As an example, in the case of frozen shoulder, nearly 30% of patients do not improve with first-line treatment methods (Shaffer et al, 1992). However, treatment options are limited for moderate MSDs, which do not necessarily respond to first-line treatments but which also do not warrant surgery. Indeed, surgery is typically reserved for only the most severe MSDs and comes with its own set of drawbacks. In particular, surgery is associated with lengthy recovery times, a risk of nosocomial infection, as well as a risk of complications from general anesthesia, in particular in the elderly.

Intra-arterial embolization represents an interesting alternative to the above therapies. While transcatheter arterial embolization (TAE) is commonly used in the treatment of tumors (e.g., for pre-operative devascularization or to deliver chemotherapeutic agents directly to a tumor) and to manage bleeding (e.g., in postpartum hemorrhage or bleeding from synovitis in patients with hemophilia (see e.g., Klamroth et al., 2009)), it is only more recently that this technique has been evaluated in the context of treating MSDs.

The interest in applying embolization techniques to MSDs arised from the observation that affected tissues, such as tendons and entheses, undergo increased neovascularity when injured (see e.g., Alfredson et al., 2003). In the case of joint-related MSDs, inflammation may induce synovial angiogenesis, leading to a redistribution of the vessels within the synovium (Bonnet and Walsh, 2005). Okuno et al. initially explored the possibility of treating refractory tendinopathy or enthesopathy with an embolizing composition composed of a mixture of imipenem (an antibiotic of the carbapenem family), cilastatin sodium (an enzyme inhibitor which prevents its breakdown into nephrotoxic metabolites), and Hexabrix^{®} as an iodinated contrast agent, administered by intra-arterial injection (2013). Although only a small number of patients were initially tested, preliminary results showed that intra-arterial embolization could relieve unrelenting pain associated with refractory tendinopathy or enthesopathy (Okuno et al., 2013). However, patients may notably be allergic to imipenem and/or to cilastatin sodium. In addition, the use of imipenem/cilastatin sodium is authorized solely for the treatment of bacterial infections in many countries. Of note, the French Authority tasked with the evaluation of health products from a medical and economic perspective (HAS) and the European Medicines Agency (EMA) recommend restricting the use of imipenem as much as possible, in particular to severe infections with drug resistant enterobacteria, to limit development of antibiotic resistance. Clearly, imipenem/cilastatin sodium cannot be injected in peripheral arteries as this is associated with a high risk of potentiating antibiotic resistance in enterobacteria and *Pseudomonas aeruginosa* which are themselves responsible for clinically high-risk septicemia. Thus, imipenem/cilastatin sodium is not a viable treatment option for MSDs in the clinic.

International application WO2020/231842 suggested using an embolic material comprising a plurality of spherical embolization beads for embolizing a hypervascular vessel formed in response to chronic inflammation.

More recently, calibrated microspheres composed of trisacryl crosslinked with gelatin (Embosphere^{®}, Merit Medical) have been administered via geniculate artery embolization in clinical trials to evaluate the safety and efficacy in treating refractory knee pain associated with knee osteoarthritis (Isaacson and Bagla, 2018). Preliminary results showed decreased pain in the majority of patients. However, microspheres are non-resorbable and cause permanent occlusion in contrast to the product comprising a mixture of imipenem and of cilastatin sodium. Indeed, when treating MSDs, temporary occlusion is preferred, as this allows for vessel recanalization while reducing the risk of permanent tissue ischemia. In addition, microspheres have been shown to be associated with an increased risk of adverse events. In particular, of 20 patients treated with polymethylmethacrylate (Embozene) microspheres, 16 minor complications were identified (Bagla et al., 2020). Furthermore, such particulate embolic agents are disadvantageous as they are not easily targeted in the vessel and are not radio-opaque, making it difficult to visualize them under fluoroscopy / fluorography, unless they are soaked in a contrast agent prior to injection. In addition, forceful injection of calibrated particles through a microcatheter or their dehydration may also result in sphere cracking.

In this context, new embolizing compositions are needed for the treatment of MSDs. Such compositions should generate only temporary embolization, i.e. an embolization lasting for less than 30 days, and thus limit the risk of ischemia. Such compositions should be relatively inexpensive and widely available. In particular, such compositions should be available for the indicated use, rather than reserved for specific medical conditions, such as treatment of bacterial infection.

### Description

In the context of the present invention, the inventors surprisingly found that a composition comprising an iodinated oil and an aqueous phase comprising a water-soluble contrast agent could be used to treat inflammatory hypervascularization associated with a musculoskeletal disorder. Indeed, embolization in a minipig model of osteoarthritis led to an immediate disappearance of inflammatory hypervascularization in all animals tested. In addition, the embolizing emulsion of the present invention advantageously has a greater embolizing capacity than that of other compositions (e.g., microspheres) and is available at lower cost.

Thus, in a first aspect, an embolizing emulsion comprising an iodinated oil and an aqueous phase comprising a water-soluble contrast agent for use in the treatment of inflammatory hypervascularization associated with a musculoskeletal disorder is provided herein.

The term "embolizing" refers to a process wherein a material is injected into a blood vessel which thereafter at least partially fills or plugs the blood vessel so that blood flow through the vessel is reduced or ceases. Thus, the term "embolizing emulsion" as used herein refers to a composition which forms a partial or complete embolism or blockage upon application to non-enteral bodily fluids, usually the blood. In one aspect, an embolizing emulsion may reduce blood flow (e.g., by at least 50%, 60%, 70%, 80%, 90%, or 95% as compared to blood flow before embolization). This may be particularly advantageous when large blood vessels or arteries are targeted. Alternatively, an embolizing emulsion may induce a complete embolization or blockage. Preferably, the embolizing emulsion provided herein induces a complete embolization. In the present case, the blockage is temporary. Indeed, iodinated oils are known to induce a transient embolization of arterial circulation. The embolizing emulsion is injected into an artery where a block or occlusion is desired. Thus, the embolizing emulsion is in liquid form at the time of injection. In the case of liquid embolics, the distance between end of the microcatheter where the liquid embolic exits the microcatheter and the target lesion can vary according to the nature of embolic agent, of the flow condition, of the viscosity of the liquid embolic, of the size of the vessels, and/or of the strength of the manual injection of the liquid embolic.

The embolization may be selective or non-selective. "Selective embolization" refers to an embolization in which the microcatheter is positioned directly in the artery responsible for hypervascularization in order to deliver the embolizing emulsion. "Non-selective" or "shower" embolization refers to an embolization in which the microcatheter is positioned proximally (i.e. at a distance) to the artery responsible for hypervascularization in order to deliver the embolizing emulsion.

Said emulsion may be an "oil-in-water" emulsion or a "water-in-oil" emulsion.

A "water-in-oil" emulsion (also referred to as an "inverse" or "W/O" emulsion) is a dispersion of droplets of an aqueous phase in a lipid phase. An "oil-in-water" emulsion (also referred to as a "direct" or "O/W" emulsion) is a dispersion of lipid phase droplets in an aqueous phase. The term "lipid phase" as used herein refers to a phase of any non-polar, organic liquid that is immiscible with water, usually an oil. Thus, the lipid phase preferably comprises an oil, more preferably an iodinated oil. Even more preferably, the lipid phase consists of an oil, more preferably an iodinated oil. The "aqueous phase" refers to the water-based phase of the biphasic mixture. The aqueous phase comprises a water-soluble contrast agent. Thus, the aqueous phase comprises water and a water-soluble contrast agent. In some cases, the aqueous phase may further comprise water-soluble additives, such as buffers, pH adjusting agents, antioxidants, tonicity/osmotic modifying agents, and/or stabilizers.

Said emulsion is preferably a water-in-oil emulsion. Thus, the present invention preferably relates to an embolizing water-in-oil emulsion comprising iodinated oil and an aqueous phase comprising a water-soluble contrast agent for use in the treatment of inflammatory hypervascularization associated with a musculoskeletal disorder.

The "iodinated oil" as provided herein comprises or consists of derivatives of iodinated fatty acids, preferably ethyl esters of iodinated fatty acids, more preferably ethyl esters of iodinated fatty acids of poppy oil, olive oil, rapeseed oil, peanut oil, soybean oil or walnut oil, even more preferably ethyl esters of iodinated fatty acids of poppy oil or of olive oil. The term "fatty acid" refers to saturated or unsaturated aliphatic carboxylic acids having a carbon-based chain of at least 4 carbon atoms. Natural fatty acids have a carbon-based chain of 4 to 28 carbon atoms (usually an even number). A "long-chain fatty acid" refers to a length of 14 to 22 carbon atoms and the term "very-long-chain fatty acid" refers to a length of more than 22 carbon atoms. In contrast, a "short-chain fatty acid" refers to a length of 4 to 10 carbon atoms, such as 6 to 10 carbon atoms, in particular 8 or 10 carbon atoms. The skilled person is well aware of the associated nomenclature and in particular uses: Ci-Cp to denote a range of Ci to Cp fatty acids, and Ci + Cp to indicate the total of the Ci and Cp fatty acids. As an example, fatty acids having 14 to 18 carbon atoms are written as "C14-C18 fatty acids;" the total of C16 fatty acids and C18 fatty acids is written as C16+C18; for a saturated fatty acid, a skilled person will use the following nomenclature Ci: 0, where i is the number of carbon atoms of the fatty acid (palmitic acid will thus be designated by the nomenclature (C16:0)); for an unsaturated fatty acid, a skilled person will use the following nomenclature Ci: x n-N where N will be the position of the double bond in the unsaturated fatty acid starting from the carbon opposite the acid group, i is the number of carbon atoms of the fatty acid, and x is the number of double bonds (unsaturations) of this fatty acid (oleic acid will thus be designated by the nomenclature (C18:1 n-9).

Most preferably, the iodinated oil comprises a mixture of ethyl esters of iodinated and non-iodinated fatty acids of poppyseed oil. Alternatively, the iodinated oil consists of a mixture of ethyl esters of iodinated and non-iodinated fatty acids of poppyseed oil.

Poppy oil is preferably obtained from *Papaver somniferum* var. *nigrum* (also known as black poppy) and more preferably from the seeds of *Papaver somniferum* var. *nigrum.* The poppy oil, also known as poppyseed oil, preferably contains more than 80% of unsaturated fatty acids (in particular of linoleic acid (C18:2 n-6) and oleic acid (C18:1 n-9)), of which at least 70% is linoleic acid and at least 10% is oleic acid. The iodinated oil is obtained from iodination, preferably complete iodination, of an oil such as poppy oil under conditions which allow bonding of one iodine atom for each double bond of the unsaturated fatty acids (Wolff, 2001) followed by transesterification.

The iodinated oil according to the invention preferentially contains from 29% to 53% (w/w), more preferentially from 37% to 39% (w/w), of iodine. The iodinated oil of the emulsion is in the lipid phase of the emulsion.

As examples of iodinated oils, mention may be made of Lipiodol^{®} (a mixture of ethyl esters of iodinated fatty acids of poppy oil, also referred to as ethiodized oil), Brassiodol^{®} (derived from rapeseed (*Brassica compestis*) oil), Yodiol^{®} (derived from peanut oil), Oriodol^{®} (derived from poppy oil but in the form of fatty acid triglycerides) and Duroliopaque^{®} (derived from olive oil).

Preferably, the iodinated oil is a mixture of ethyl esters of iodinated and non-iodinated fatty acids of poppyseed oil (i.e. ethiodized oil, also known as Lipiodol^{®}). Lipiodol^{®} is a pale yellow/amber-colored iodinated oil commercialized by Guerbet. It is used in particular for visualization, localization and/or vectorization during transarterial chemoembolization of hepatocellular carcinomas at the intermediate stage in adults, as well as for selective hepatic arterial diagnosis of the extension of hepatic malignant lesions. This oil mainly contains (in particular, more than 84%) a mixture of ethyl esters of long-chain iodinated fatty acids (in particular C18 fatty acids) derived from poppyseed oil, preferably of a mixture of ethyl monoiodostearate and ethyl diiodostearate. The iodinated oil may also be an oil based on a monoiodinated ethyl ester of stearic acid (C18:0) derived from olive oil (e.g., Duroliopaque^{®}, mentioned above).

The main characteristics of Lipiodol^{®} are provided in the table below:

| **Compounds** | **Proportion in the fatty acid mixture** |
|---|---|
| Ethyl palmitate (Ethyl C16:0) | 4.6% to 6.7% (w/w), preferably 4.8% (w/w) |
| Ethyl stearate (Ethyl C18:0) | 0.8% to 1.9% (w/w), preferably 1.2% (w/w) |
| Ethyl monoiodostearate | 11.3% to 15.3% (w/w), preferably 13.4% (w/w) |
| Ethyl diiodostearate | 73.5% to 82.8% (w/w), preferably 78.5% (w/w) |

Additional characteristics of Lipiodol^{®} are provided in the table below:

| | |
|---|---|
| **Iodine content** | 37% to 39% (w/w) (e.g. 480 mg/ml) |
| **Viscosity at 37°C** | 25 mPa.s |
| **Viscosity at 20°C** | 50 mPa.s |
| **Density** | 1.268 to 1.290 g/cm³ at 20°C, preferably 1.28 g/cm³ |

Preferably, the amount of iodinated oil present in the emulsion according to the invention does not exceed 15 ml. Preferably, the lipid (or oil) phase consists essentially of iodinated oil, more preferably the lipid phase consists of iodinated oil as defined herein.

The density of the lipid phase of the emulsion is preferably from 1.10 to 1.30, more preferably from 1.20 to 1.30, even more preferably of 1.28.

The term "water-soluble contrast agent" as used herein refers to a biocompatible (non-toxic) material capable of being monitored (e.g., by radiography) when injected into a subject, and which is soluble in water. The water-soluble contrast agent is preferably radio-opaque and can thus be detected by radiography (e.g., on an angiogram or arteriogram). It may notably comprise barium sulfate or iodine.

Preferably, the water-soluble contrast agent is an iodinated contrast agent. Thus, it contains iodine. In the iodinated contrast agent, the iodine may be bound to either an ionic compound or to an organic compound, in which case it is classified as a "non-ionic" compound. Examples of ionic iodinated contrast agents include meglumine diatrizoate (Hypaque^{®}), metrizoate (Isopaque^{®}), iothalamate (Conray^{®}), and ioxaglate (Hexabrix^{®}), meglumine amidotrizoate and meglumine ioxitalamate. Examples of non-ionic iodinated contrast agents include iobitridol (Xenetix^{®}), iopamidol (Iopamiron^{®}, Isovue^{®}), iomeprol (Iomeron^{®}), ioversol (Optiray^{®}, Optiject^{®}), iohexol (Omnipaque^{®}), iopentol (Imagopaque^{®}), ioxitol (Oxilan^{®}), iopromide (Ultravist^{®}), metrizamide (Amipaque^{®}), iosarcol (Melitrast^{®}), iotrolan (Isovist^{®}), iodixanol (Visipaque^{®}), iosimenol and iosimide (Univist^{®}). The iodinated contrast agent may be in monomeric or dimeric form. It may have high osmolality (i.e. 1200 mOsm/kg or higher), low osmolality (i.e. 500-900 mOsm/kg), or be iso-osmolar (i.e. approximately 290 mOsm/kg).

Preferably, the water-soluble iodinated contrast agent is non-ionic. Preferably, the water-soluble iodinated contrast has low osmolarity (i.e. from 500 to 900 mOsm/kg) or is iso-osmolar. Preferably, the water-soluble iodinated contrast agent comprises ioversol, iopamidol, iomeprol, iopromide, iohexol, iobitridol, and iodixanol or a mixture of two or more thereof. More preferably, the water-soluble iodinated contrast agent is selected from the group consisting of ioversol, iopamidol, iomeprol, iopromide, iohexol, iobitridol, and iodixanol, even more preferably iobitridol and ioversol, most preferably ioversol.

The ratio of the lipid phase:aqueous phase (in other words, the ratio of iodinated oil:aqueous phase comprising the water-soluble contrast agent as provided herein) is preferably at least 2:1 v/v such that an oil-in-water composition is obtained. Indeed, a 1:1 v/v ratio between the lipid phase and the aqueous phase naturally favors an O/W direction. In cases where a water-in-oil emulsion is desired, the amount of iodinated oil added must be increased. A ratio of at least 2:1 v/v of iodinated oil to the aqueous phase (also expressed as the ratio of iodinated oil:aqueous phase or lipid phase:aqueous phase) makes it possible to obtain a W/O emulsion. More preferably, the ratio of iodinated oil:aqueous phase is at least 3:1 v/v or at least 4:1 v/v. In one aspect, the ratio (v/v) of the iodinated oil:aqueous phase is comprised between 2:1 and 4:1, more particularly comprised between 5:2 and 10:3. The ratio (v/v) of the iodinated oil:aqueous phase may notably be equal to 3:1 or 4:1.

The concentration of the water-soluble contrast agent (e.g., ioversol) in the aqueous phase (i.e. in water) may range from 500 to 750 mg/mL, more particularly from 509 to 741 mg/mL. In particular, the concentration of the water-soluble contrast agent (e.g., ioversol) in the aqueous phase may be 509, 636, 678 or 741 mg/mL. Preferably, the concentration of iodine in the aqueous phase ranges from 240 to 400 mg iodine/mL (mg I/mL), from 240 to 350 mg I/mL, or from 240 to 320 mg I/mL). In particular, the concentration of iodine in the aqueous phase may be 240, 300, 320, 350, 370 or 400 mg I/mL. Iodine present in the aqueous phase derives from an iodinated water-soluble contrast agent, and the above indicated ranges of iodine concentration correspond to the above indicated ranges of concentration of the water-soluble contrast agent, when using conventional water-soluble contrast agents as disclosed above, in particular ioversol.

The lipid and aqueous phases may have the same density (in other words, they are of equal density) or different densities. loversol is one preferred iodinated contrast agent; Optiray^{®} 240, Optiray^{®} 300, Optiray^{®} 320, Optiray^{®} 350 have densities of 1.281, 1.352, 1.371 and 1.405, respectively. Iobitridol (Xenetix^{®}) is another preferred iodinated contrast agent. Xenetix^{®} 250 and Xenetix^{®} 300 products have densities of 1.28 and 1.34, respectively. Preferably, the lipid and aqueous phases have the same density (in other words, they are of equal density) or similar densities (i.e. they differ by no more that 10% from one another).

The embolizing emulsion may have a viscosity at 20°C ranging from 70 to 200 mPa.s, preferably from 120 to 170 mPa.s, more preferably from 150 to 165 mPa.s, even more preferably from 150 to 165 mPa.s, and/or a viscosity at 37°C ranging from 40 to 140 mPa.s, preferably from 70 to 140 mPa.s, more preferably from 80 to 130 mPa.s, or even more preferably from 90 to 130 mPa.s. Viscosity values may notably be obtained using a Malvern Instruments Kinexus Pro rheometer, having a 4° cone-plate cell with a diameter of 40 mm. Measurements are carried out at an imposed stress in a range of from 0.16 to 10 Pa.

As a non-limiting example, the size of the droplets in the emulsion (i.e. the aqueous phase droplets) may range from 1 to 500 µm, from 1 to 200 µm, or from 5 to 150 µm. Preferably, emulsion droplet size ranges from 10 to 100 µm, more preferably from 10 to 80 µm, or even more preferably from 30 to 80 µm. Size may vary from one droplet to the next, so long as they are within the desired size range. Preferably, the aqueous phase droplets are uniformly distributed. Droplet size and uniformity can be measured using an optical microscope (for example, the Leica DM2000 LED microscope). If aggregates of droplets are observed, these droplets are not uniformly distributed.

The aqueous phase may further comprise one or more water-soluble additives. Said one or more additives may notably be a buffer, a pH adjusting agents, an antioxidant, a tonicity/osmotic modifying agent, a density modifying agent and/or a stabilizer. Said additive(s) notably do not have any therapeutic effect in the body. Preferably, the aqueous phase comprises a buffer, more preferably tromethamine. Preferably, the aqueous phase comprises a stabilizer, more preferably disodium calcium edetate. Preferably, the pH of the aqueous phase is adjusted to a pH from 6.0-7.2, preferably with hydrochloric acid or sodium hydroxide.

While the embolizing emulsion provided herein may comprise additional additives such as those described above, it preferably does not comprise any additional "active" ingredients. By "active ingredient" is meant herein a component having a therapeutic activity in the body.

A first example of an active ingredient that is preferably not present in the embolizing emulsion is a chemotherapeutic anticancer agent. Indeed, as the emulsion is used in the treatment of inflammatory hypervascularization associated with a musculoskeletal disorder, the presence of such agents would be nefast. A non-limiting example of such chemotherapeutic anticancer agents includes anthracyclines, mitomycin C, platinum complexes, radioelements, alkylation/amine methylation agents, anti-mitotic agents/tubulin inhibitors, topoisomerase inhibitors, pyrimidine antagonists, guanidine antagonists, folic acid antagonists, etc. More particularly, the chemotherapeutic anticancer agent excluded from the present emulsion is doxorubicin, epirubicin, idarubicin, nemorubicin, mitoxantrone, pirarubicin, paclitaxel, cisplatin, carboplatin, oxaliplatin, lobaplatin, cyclophosphonamide, mitomycin C, fotemustine, irinotecan, mitoxantrone, everolimus, sorafenib, 5-fluorouracil, methotrexate, gemcitabine, carmustine, dacabazine, etoposide, vinorelbine, topotecan, estramustine, and any combination thereof.

Thus, the embolizing emulsion preferably does not comprise a chemotherapeutic anticancer agent. More preferably, the embolizing emulsion does not comprise any of the above-listed chemotherapeutic anticancer agents.

A further example of an active ingredient that is preferably not present in the embolizing emulsion is nanoparticles or polymeric particles, more preferably embolic particles. Indeed, while nanoparticles or polymeric particles may be used as alternative embolizing agents they are not desired in the context of the present invention. In particular, non-resorbable nanoparticles or polymeric particles cause permanent occlusion which can result in ischemia. Resorbable nanoparticles or polymeric particles may cause inflammation and/or thrombus formation as a result of their partial degradation and the distal migration of particles or fragments. Thus, they are not desired in the context of the present invention. The term "nanoparticle" as used herein refers to a solid particle having an average diameter of between about 1 nm and about 500 nm. More particularly, the embolizing emulsion does not comprise polyester-based nanoparticles (e.g., polyacetic acid polylactide-based nanoparticles, poly glycolic acid (polyglycolide), lactide-glycolide copolymers, copolymers of lactide-glycolide-co-polyethylene glycol, polyortho-esters, polyanhydrides, polybutylacetone, polyvalerolactone, poly malic acid, polylactones). The term "polymeric particle" as used herein refers to a particle that comprises one or more types of polymer (e.g. polyesters (e.g., poly[hydroxy acids], poly[cyclic esters], etc.), polycarbonates, polyorthoesters, polyanhydrides, polycyanoacrylates (e.g., polyalkylcyano-acrylate or "PACA") and polyphosphazines). A polymeric particle may be a nanoparticle.

The term "embolic particle" refers to any solid or non-dissolved substance that forms an embolism or blockage upon application to non-enteral bodily fluids, usually the blood. An embolic particle may more particularly comprise nanoparticles and/or embolic particles. The particles may be spheroid or oblong or have irregular geometry. In contrast, the embolizing emulsion provided herein is composed of liquid substances (e.g., iodinated oil and an aqueous phase comprising a water-soluble contrast agent).

Thus, the embolizing emulsion also preferably does not comprise nanoparticles or polymeric particles. Even more preferably, the composition does not comprise any embolic particle.

The term "musculoskeletal disorder" or "MSD" as used herein refers to any disease or condition in which the function of the musculoskeletal system (e.g., a muscle, ligament, tendon, cartilage, bursa, joint, or bone) of a subject is impaired. The MSD may be a local or systemic disorder; it may be acute or chronic, more preferably chronic. In cases of chronic MSDs, they may be continuously present, or recur sporadically (e.g., flares). An MSD may be due to behavioral, environmental, traumatic, immune, degenerative, and/or genetic factors and/or may be related to systemic disease. As an example, an MSD may at least partially result from repetitive motion or forceful exertions performed e.g., in the workplace or in the context of sports. Thus, in one aspect, the MSD is a sports injury. MSDs include strains, sprains, fractures, torsions, tears, inflammatory rheumatoid diseases, enthesopathies, and tendinopathies (such as tendinitis and tenosynovitis). MSDs may notably affect the elbow, knee, wrist, shoulder, hip, heel, ankle, thumb, or spine (more particularly the cervical, dorsal, or lombo-sacral spine).

The term "inflammatory rheumatoid disease" or "inflammatory rheumatic disease" refers to diseases which produce acute or chronic inflammation in particular in the joints, though other tissues, such as muscle or connective tissue, may also be affected. Inflammation may be localized and/or systemic, and in many cases, arise as a result of an autoimmune disorder. As a non-limiting example, inflammatory rheumatoid diseases include osteoarthritis (such as knee or hip osteoarthritis), cervical spondylosis, rheumatoid arthritis (RA), acute crystal arthritis (e.g., gout), spondyloarthropathies (ankylosing spondylitis (AS), and psoriatic arthritis (PsA)), Sjogren's syndrome, scleroderma, infectious arthritis, plantar fasciosis, juvenile idiopathic arthritis, polymyalgia rheumatica, fibromyalgia, lupus, and vasculitis.

The term "enthesopathy" refers to a disorder involving the attachment of a tendon or ligament to a bone. Enthesopathies notably comprise Achilles tendon enthesopathy, epicondylitis, ankylosing spondylitis, Plantar fasciitis, rotator cuff syndrome of shoulder and allied disorders, enthesopathy of elbow region, enthesopathy of wrist and carpus, olecranon bursitis, prepatellar bursitis, bursitis of hand or wrist, enthesopathy of hip region, bursitis of hip (e.g., trochanteric bursitis, iliopsoas bursitis), enthesopathy of knee, enthesopathy of ankle, enthesopathy of tarsus, and enthesopathy of calcaneus (e.g., inferior calcaneal bursitis).

The term "tendinopathy" refers to a disorder or injury of a tendon. Tendinopathies notably comprise shoulder tendinitis, calcific tendinitis (rotator cuff tendinitis), Achilles tendinitis, biceps tendinitis, quadriceps tendinosis, lateral epicondylitis (tennis elbow), medial epicondylitis (golfer's elbow), De Quervain's tenosynovitis, stenosing tenosynovitis (trigger finger/thumb), wrist tenosynovitis and patellar tendinopathy.

As a further example, MSDs also include neck tension, osteoporosis, Baker's cysts, adhesive capsulitis (frozen shoulder), carpal tunnel syndrome, tarsal tunnel syndrome, radial tunnel syndrome, hand arm vibration syndrome, knee meniscus injury, degenerative disc disease, ruptured/herniated disc, digital neuritis, thoracic outlet compression syndrome, Dupuytren's Contracture, sesamoiditis, lyme disease, and the like.

Preferably, the MSD disorder affects the elbow, the knee, the wrist, the shoulder, the hip, the heel, the ankle, the thumb, and/or the spine. When said MSD affects the spine, it more preferably affects the cervical, dorsal, or lombo-sacral spine.

Preferably, the MSD is selected from enthesopathies, tendinopathies, inflammatory rheumatoid diseases and carpel tunnel syndrome. More preferably, the MSD is:
- an enthesopathy selected from Achilles tendon enthesopathy, epicondylitis, ankylosing spondylitis, Plantar fasciitis, rotator cuff syndrome of shoulder, enthesopathy of elbow region, enthesopathy of wrist and/or carpus, olecranon bursitis, prepatellar bursitis, hand or wrist bursitis, enthesopathy of hip region, hip bursitis, enthesopathy of knee, enthesopathy of ankle, enthesopathy of tarsus, and enthesopathy of calcaneus,
- a tendinopathy selected from shoulder tendinitis, calcific tendinitis, Achilles tendinitis, biceps tendinitis, quadriceps tendinosis, lateral epicondylitis, medial epicondylitis, De Quervain's tenosynovitis, stenosing tenosynovitis, wrist tenosynovitis, patellar tendinopathy, or
- an inflammatory rheumatoid disease selected from osteoarthritis, cervical spondylosis, rheumatoid arthritis (RA), acute crystal arthritis, spondyloarthropathies, psoriatic arthritis), Sjogren's syndrome, scleroderma, infectious arthritis, plantar fasciosis, juvenile idiopathic arthritis, polymyalgia rheumatica, fibromyalgia, lupus, vasculitis.

Even more preferably, the MSD is selected from tennis elbow, adhesive capsulitis, osteoarthritis, syndrome, or Achilles tendinitis.

The MSD may be resistant to one or more treatments (e.g., first-line treatments, e.g. pharmacological treatments such as oral analgesics, anti-inflammatory medication, corticosteroid injections, or physical therapy). MSDs that are "resistant" to treatment either do not respond to treatment and/or have a reduced ability to produce significant responses (e.g., a partial and/or complete response) with treatment at a maximum recommended dosage, duration, and/or frequency. Resistance may be acquired (i.e. it develops over time), in particular when a chronic MSD requires continuous treatment. As a particular example, pain is opiate-resistant when inadequate analgesia is achieved at levels of opiate therapy which cause intolerable side effects. In some cases, MSD is refractory. The term "refractory" as used herein refers to an MSD which does not respond to at least two different pharmacological therapies (such as those mentioned above). Said at least two different therapies preferably have different mechanisms of action.

Preferably, the MSD is resistant or refractory to treatment with oral analgesics, anti-inflammatory medication, physical therapy, and/or corticosteroid injections.

A further aspect of the present invention relates to an embolizing emulsion as provided herein for use in the treatment of inflammatory hypervascularization associated with a musculoskeletal disorder wherein said treatment comprises administering the embolizing emulsion to at least one target artery supplying blood to the affected tissue(s). The embolizing emulsion of the present invention causes the formation of a temporary embolism. Preferably, embolization of the targeted artery(ies) lasts for less than 24 hours. More preferably, embolization of the targeted artery(ies) lasts for 6-12 hours.

The term "inflammatory hypervascularization" as used herein refers to an increased number or concentration of blood vessels, and which is associated with inflammation. It may involve the formation of de novo vessels (i.e. neovascularization), the formation of new vessels from existing vessels (e.g. growth of new capillaries from post-capillary venules), and/or an increased diameter of existing arterial vessels (i.e. arteriogenesis).

While not part of the claimed invention, it is also disclosed herein the use of the embolizing emulsion as provided herein for the manufacture of a medicament for treating inflammatory hypervascularization associated with a musculoskeletal disorder.

While not part of the claimed invention, the present disclosure also describes the use of the embolizing emulsion as provided herein in the treatment of inflammatory hypervascularization associated with a musculoskeletal disorder.

While not part of the claimed invention, a method for the treatment of inflammatory hypervascularization associated with a musculoskeletal disorder in a subject in need thereof is further disclosed herein. More particularly, a method of treating inflammatory hypervascularization associated with a musculoskeletal disorder in a subject in need thereof comprises the administration of a therapeutically effective amount of the embolizing emulsion as provided herein.

The term "treatment" as used herein refers to an alleviation of symptoms of an MSD (i.e. associated with inflammatory hypervascularization), or inhibition of further progression or worsening of said symptoms. The term "treatment" as used herein may also refer to a delay in the appearance of symptoms, a reduction in symptom severity upon appearance, or a reduction in the frequency of disease episodes. Similarly, the term an "effective amount" as used herein in the context of the embolizing emulsion, or a "therapeutically effective amount" of the embolizing emulsion refers to an amount of the agent that alleviates, in whole or in part, symptoms associated with the MSD, or halts or slows further progression or worsening of those symptoms. In particular, an "effective amount" refers to an amount which is effective in achieving the desired therapeutic result. A therapeutically effective amount may notably be administered in one or more administrations, in particular wherein each administration occurs at a different site within an affected area or in different affected areas (e.g., at different joints in the case of generalized osteoarthritis). In particular, a therapeutically effective amount of the embolizing emulsion is administered in one, two, three or more injections over a given period of time and/or to different arteries (e.g., located at the same joint or in different joints). A therapeutically effective amount is also one in which any toxic or detrimental effects of compounds of the invention are outweighed by the therapeutically beneficial effects.

A "subject" refers to any mammal, and may more particularly be any human individual, regardless of their age. Specifically, the subject may be a human adult or child. The term "adult" refers herein to an individual of at least 16 years of age. The term "child" comprises infants from 0-1 years of age and children from 1-8 years of age, 8-12 years of age, and 12-16 years of age.

While not part of the claimed invention, a method for the treatment of inflammatory hypervascularization associated with a musculoskeletal disorder in a subject in need thereof, wherein said inflammatory hypervascularization affects one or more tissue(s) of the subject, comprising:
- providing an embolizing composition comprising iodinated oil and an aqueous phase comprising a water-soluble contrast agent, and
- temporarily embolizing at least one artery supplying blood to the affected tissue(s) with the embolizing composition, preferably via transcatheter arterial embolization
is further disclosed herein.

While not part of the claimed invention, the present disclosure also describes a kit comprising the iodinated oil and a water-soluble contrast agent as provided herein, and instructions for administering the immunogenic or immunotherapeutic composition described herein to a subject. The water-soluble contrast agent may be provided either in lyophilized form or in an aqueous phase. The iodinated oil and water-soluble contrast agent may be provided in separate containers. In this case, the embolizing emulsion may be prepared by mixing the iodinated oil with the aqueous phase comprising the water-soluble contrast agent prior to administration. Alternatively, the iodinated oil and aqueous phase may be provided in the same container (e.g., as an emulsion which may be administered directly). In this case, the emulsion further comprises a stabilizing agent.

### Figures

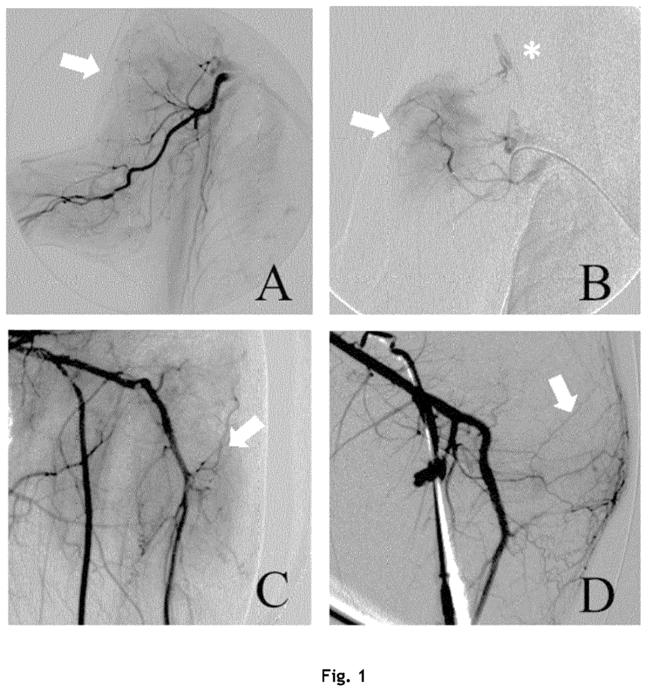

**Figure 1****. Swine model of osteoarthrosis.** (A, B) Representative angiographies of the shoulder 7 days after alcohol injection showing hypervascularization (arrow) and early venous return (*). (C, D) Examples of angiography of untreated shoulder 20 days after alcohol injection showing persistent hypervascularization (arrow).

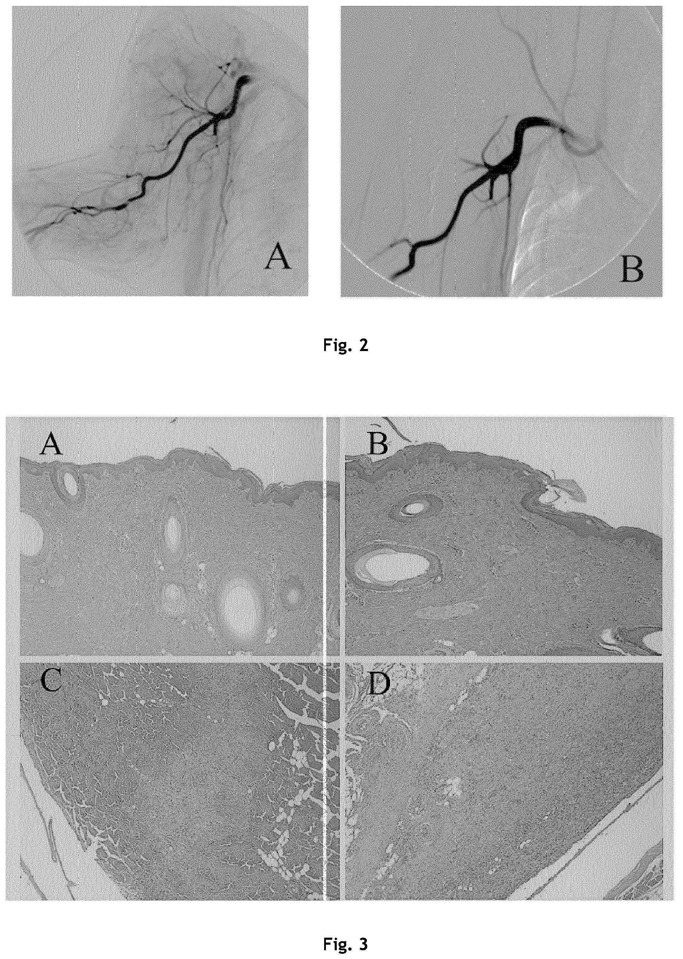

**Figure 2****. Effect of embolizing emulsion in swine model of osteoarthrosis.** Representative comparison of angiography at baseline (A) and post embolization with Lipiodol^{®} emulsion (B) showing no signs of hypervascularization.
**Figure 3****. Histological staining of untreated (control) limbs as compared to limbs treated with the embolizing emulsion.** (A) Untreated skin, (B) Treated skin, (C) Untreated tendon, (D) Treated tendon.
**Figure 4****. Illustration of embolization persistence with an Lipiodol^{®}/loversol emulsion.** (A) Exemplary angiographies performed over time for Pig #1 with administration of LUF/Optiray^{®} to the left shoulder. (B) Exemplary angiographies performed over time for Pig#2 with administration of LUF/Optiray^{®} to the inferior polar artery of the left kidney.
**Figure 5****. Effect of joint embolization in a clinical trial.** (A) Pain and (B) functional impairment were evaluated over time in 6 patients with knee osteoarthritis.

### Examples

The following examples are included to demonstrate preferred embodiments of the invention. All subject-matter set forth or shown in the following examples and accompanying drawings is to be interpreted as illustrative, and thus not in a limiting sense. The following examples include any alternatives, equivalents, and modifications that may be determined by a person skilled in the art.

### 1. Materials and methods

### 1.1 Osteoarthritis model

Four minipigs weighing 17-25 kg were kept in separate cages in an environmentally controlled animal research facility. Food and water were provided ad libitum.

All procedures were performed under general anesthesia with a mixture of ketamine (50 mg/kg) and xylazine (5 mg/kg) IM. Minipigs were ventilated through a mask at a rate of 20 breaths per minute and a tidal volume of 45-50 ml using room air enriched with oxygen.

### 1.1.1 Preparation of the embolizing emulsion

Lipiodol^{®} emulsions were formulated by repetitive back-and-forth pumping of two 20-ml syringes through a 3-way stopcock using 3 cc of ioversol contrast agent (Optiray^{®} 240, Guerbet, France) and 9 cc of Lipiodol^{®} (Guerbet, France).

### 1.1.2 Osteoarthrosis swine model

A direct puncture of the upper right shoulder and lower bilateral knee was performed under fluoroscopy using a standard 18 G catheter (Angiocath, BD medical, Utah, USA). An injection of 5 ml pure ethanol (100%) was performed in the right shoulder and both knee joints to induce chronic inflammation. The left shoulder joint was used as control.

7 days after model creation, a puncture of the right femoral artery was performed under ultrasound guidance followed by a selective angiography of bilateral subclavian and femoral artery using a 4 Fr Cobra catheter (Terumo, Tokyo, Japan) in order to evaluate the presence of hypervascularization in the 4 joints.

After selective catheterization of the right subclavian artery, a superselective catheterization of the feeding hypervascular area was performed using a 2.7 Fr microcatheter (Progreat^{®}, Terumo, Tokyo, Japan). Complete embolization was obtained by the injection of the Lipiodol^{®} emulsion until stasis (selective embolization; right shoulder). In addition, catheterization of the right femoral artery was performed and a non-selective ("shower") embolization was performed from the Cobra catheter (non-selective embolization; right knee). After embolization, an angiographic control was performed to evaluate the immediate technical success.

No embolization was performed in the left knee (positive control for the model). Hemostasis was obtained by manual compression of the puncture site and the animal was awakened.

14 days after embolization, a selective control angiography was performed in subclavian and femoral arteries to evaluate the patency of the parent vessel at follow-up via a left common femoral approach. Minipigs were sacrificed thereafter.

### 1.1.3 Histological evaluation

Immediately after sacrifice, enthesis and tendon samples were obtained from the 4 joints and fixed in 10% formaldehyde for histological analysis. Samples were processed according to standard procedures, embedded in paraffin, and sectioned. Specifically, each section was stained with hematoxylin and eosin (H&E). An independent pathologist analyzed the samples and characterized the lesions. The pathologist was blinded to the treatment received by the minipig.

### 1.1.4 End points of the study

The presence of hypervascularization associated with histological signs of sub-acute inflammatory disease confirmed the successful creation of the animal model.

The primary efficacy endpoint was the successful embolization of the target vessel with disappearance of inflammatory hypervascularization at the immediate post-embolization angiography.

The primary safety endpoint was met when all of the following criteria were observed: patency of the parent vessel at the angiographic control 14 days after embolization, absence of non-target embolization, and absence of joint and skin necrosis.

### 1.1.5 Statistics

All data were analyzed by SPSS 20.0 (SPSS Inc., Chicago, IL, USA). Categorical variables were expressed as absolute values and percentages and compared with the chi-squared test. Numerical variables were expressed as mean value ± standard deviation and compared with the paired Student t-test. p<0.05 (2-sided) was considered statistically significant in all analyses.

### 1.2 Evaluation of characteristics of a Lipiodol^{®}/loversol emulsion

Two 45 kg healthy pigs were anesthetized and injected intra-arterially via an interventional procedure using a fluoroscope. An interventional procedure of embolization is a minimally invasive procedure that is performed under image guidance (X-ray in this case with the use of a fluoroscope) and in which catheters are used for administration of an embolizing product.

Stability was evaluated for two different products:
1. Imipenem/Cilastatin, Mylan (IPM/CS)
   To prepare the suspension, 500 mg of IPM/CS powder was suspended in 10 ml of loversol contrast media (Optiray^{®} 300, Guerbet). The suspension was placed in a 10 mL syringe and branched through a 3-way stopcock to a 3mL syringe. The suspension was pumped 20 times to obtain a uniform suspension.
2. Lipiodol^{®} Ultra Fluid (LUF)-based emulsion with a ratio 3:1 (3 vol. Lipiodol^{®} to 1 vol. of contrast media loversol (Optiray^{®} 300)), also referred to herein as "LUF/Optiray^{®}". 6 mL of Lipiodol^{®} were placed in a 10 mL syringe branched with a 3-way stopcock to a 3 mL syringe previously filled with 2 mL of Optiray^{®} 300 (Guerbet). The solution was pumped 20 times (starting by pushing the Optiray^{®} into the Lipiodol^{®}) to obtain a homogeneous, stable emulsion.

Homogeneity/stability was evaluated by visual inspection over time.

Embolization persistence was further evaluated with the LUF/Optiray^{®} emulsion:
LUF/Optiray^{®} was injected at different target arteries as detailed in the table below.

**Table 1: Injection schema - target areas**

| | **Target area** |
|---|---|
| **Pig #1** | Inferior polar artery of the right kidney |
| | Inferior polar artery of the left kidney |
| | Left shoulder |
| **Pig #2** | Inferior polar artery of the right kidney |
| | Inferior polar artery of the left kidney |
| | Left shoulder |

| | |
|---|---|
| NA: not applicable. | |

Before embolization with LUF/Optiray^{®}, an angiography of the target area of embolization was carried out using an automatic injector to map the injection area and select the target vessel, using the following protocol: Flow rate: 4 mL/s, 8 mL of Xenetix^{®} 300 (Iobitridol, 300 mg iodine/mL; Guerbet) injected.

Embolization was then performed as follows:
- Angiography of the selected area was performed prior to embolization with approximately 1 mL Xenetix^{®} 300 (Guerbet) per injection. This image allowed the degree of repermeabilization of the embolized vessels to be determined after downstream administration of the embolizing product.
- The product (prepared as described above) was injected until complete filling of the selected area that corresponds approximatively to 1.5 mL for each embolization. The injections were carried out with a 2.4Fr DraKon^{™} microcatheter (Guerbet).
- Angiographies were performed with approximately 1 mL Xenetix^{®} 300 at 1, 2, 3, 4, 5, 6, 8, 10, 15, and 20 minutes after administration of the product.
- A CT scan was performed 1 hour after the end of all the embolization procedures to detect and evaluate persistence of embolization in the target areas of injection.

Persistence of the embolization resulting from LUF/Optiray^{®} was determined by comparing kinetics observed via angiography over time (i.e., from 1 to 20 minutes, as described above).

### 1.3 Clinical evaluation of a Lipiodol^{®}/loversol embolizing emulsion in humans

### 1.3.1 Knee osteoarthritis

Patients were seen in the clinic for painful knee osteoarthritis and considered for inclusion in a phase 1, single-arm, open-label clinical trial (ClinicalTrials.gov Identifier: NCT04733092). Inclusion criteria included:
- Diagnosis of primary inflammatory knee osteoarthritis of the target joint defined by a knee osteoarthritis according to the American College of Rheumatology (ACR) classification and a score ≥ 2 according to the classification of Kellgren and Lawrence,
- Patient not eligible for surgery (or refusing surgery),
- Analog Visual Scale (VAS) pain ≥ 40 mm despite analgesic treatment for at least 3 months,
- Failure or intolerance of treatment with NSAIDs and/or tramadol and/or acetaminophen, and/or failure or intolerance or patient refusing strong opioids medication (morphine, codeine), and
- Failure or patient refusal of corticosteroid infiltration.

6 patients were included in the trial. The knee joint was embolized with a 3:1 v/v Lipiodol^{®}/loversol emulsion and clinical examination was performed at follow-up. Pain was assessed on a Visual Analog Scale (VAS) from 0 to 100 mm, with 100 mm being the worst possible pain. Pain is expressed as a percentage of the baseline, prior to embolization. Functional impairment was assessed via a Western Ontario and McMaster Universities Arthritis Index (WOMAC) questionnaire on a scale of 0 to 96 (see McConnell et al., 2001). A higher score is indicative of worse function. Functional impairment is expressed as a percentage of the baseline WOMAC score.

### 1.3.2. Frozen shoulder

Based on the initial preclinical (pig study) and clinical (knee osteoarthritis) safety results, one patient presenting a frozen shoulder was treated by embolizing the joint with a 3:1 v/v Lipiodol^{®}/loversol emulsion.

### 2. Results

### 2.1 Osteoarthritis model in minipigs

The osteoarthritis model was successfully induced in all animals, as illustrated in Table 2, below. Indeed, all joints subjected to alcohol injection showed hypervascularization at baseline angiography and histological signs of sub-acute inflammatory disease of the joints 7 days after injection (12/12 joints, 100%). Specifically, histological samples showed signs of chronic ischemia in all joints after alcohol injection. No signs of hypervascularization or chronic inflammation were observed in negative control joints (4/4 joints, 100%).

**Table 2. Confirmation of the validity of the animal model prior to embolization**

| | Hypervascularization at baseline angiography in joints | Histological signs of chronic inflammation in joints^{a} |
|---|---|---|
| Joint to undergo selective embolization (right shoulder joint) | 4/4 | 4/4 |
| Joint to undergo non-selective embolization (right knee joint) | 4/4 | 4/4 |
| Positive control^{b} (left knee joint) | 4/4 | 4/4 |
| Negative control^{c} (left shoulder joint) | 0/4 | 0/4 |

| | | |
|---|---|---|
| a= presence of macrophages, monocytes, and lymphocytes, with the proliferation of blood vessels and connective tissue were considered signs of chronic inflammation b= positive control were the joints where an alcohol injection was performed without any embolization (left knees in our study) c= negative control were the joints where no injection of alcohol or embolization has been performed (left shoulders in our study) | | |

Successful embolization using the Lipiodol^{®} emulsion with the immediate disappearance of hypervascularization was obtained in all treated joints (8/8, 100%; see Table 3). One minipig died before the control angiography, 3 days after embolization, due to mycoplasma pneumonitis that was not related to embolization.

At the angiographic control performed 14 days after embolization, patency of the target vessel was observed in all cases (see Table 3). A reduction in hypervascularization was also observed in 5/6 joints (83.3%) with a partial reduction observed in 4/6 joints (66.7%) and a complete disappearance observed in one joint (1/6, 16.7%).

**Table 3. Safety and efficacy of embolization as determined by angiographic and histological evaluation**

| | Successful embolization^{c} of hypervascularization after immediate angiographic control | Patency of the parent artery at angiographic control 14 days after embolization | Histological signs of joint necrosis 14 days after embolization | Histological signs of skin necrosis 14 days after embolization |
|---|---|---|---|---|
| Selectively embolized joints | 4/4 | 3/3 | 0/3 | 0/3 |
| Non-selectively embolized joints | 4/4 | 3/3 | 0/3 | 0/3 |
| Positive control^{a} | NA | 3/3 | 0/3 | 0/3 |
| Negative control^{b} | NA | 3/3 | 0/3 | 0/3 |

| | | | | |
|---|---|---|---|---|
| NA= not applicable a= positive control was the joint where an alcohol injection was performed without any embolization (left knees in our study) b= negative control was the joint where no alcohol injection or embolization was performed (left shoulders in our study) c= successful embolization was defined as the disappearance of hypervascularization at immediate angiographic control | | | | |

Transient skin sign of ischemia, characterized by a rash, were observed in all 4 joints treated with non-selective embolization (4/4, 100%). These signs disappeared a few days after embolization in all cases. The pigs did not show any clinical sign of pain or deterioration in daily normal behavior. No signs of ischemia were observed in joints treated with embolization (8/8, 100%). No signs of skin or synovial necrosis were observed in histological analyses performed 14 days after embolization (see Table 3).

### 2.2 Comparison of stability of a Lipiodol^{®}/loversol emulsion as compared to Imipenem/Cilastatin

The IPM/CS suspension is homogeneous after preparation. 5 minutes after preparation, the IPM/CS powder tends to settle, showing a phase separation. Thus, the IPM/CS not stable over time. In contrast, the LUF/Optiray^{®} emulsion is stable and homogeneous after the preparation and does not show any phase separation during the entirety of the experiment. A stable emulsion guarantees a homogeneous distribution of the products in the target area with a higher therapeutic efficacy.

### 2.3 Evaluation of embolization persistence with a Lipiodol^{®}/loversol emulsion

### 2.3.1 Product radiopacity

The LUF/Optiray^{®} emulsion is detectable by fluoroscopic imaging during injection thanks to the radiopacity of the Lipiodol^{®}. After administration, Lipiodol^{®} radiopacity persisted in the vessels and was detected until 8 minutes after administration.

### 2.2.3 Embolic properties

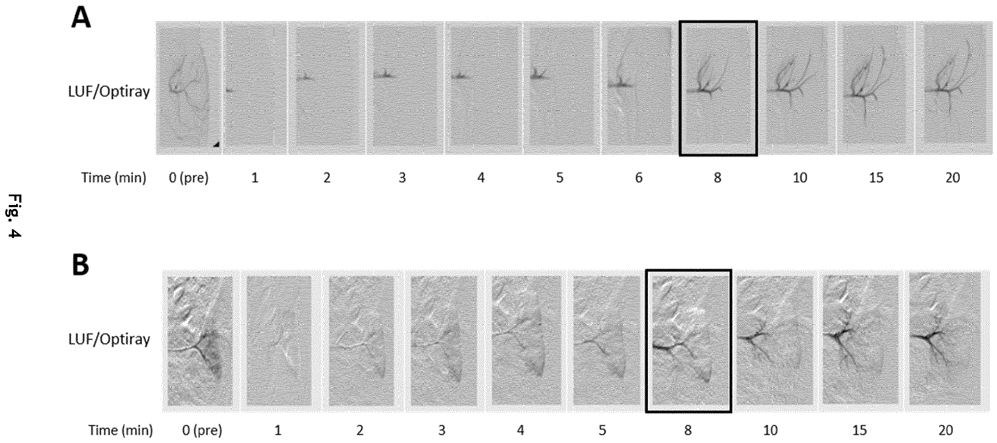

Target arteries where LUF/Optiray^{®} emulsion was injected were completely repermeabilized 8 minutes after product administration, indicating that embolization is successful and transitory. In addition, embolization was reproducible and predictable. Indeed, as illustrated in Figure 4, Xenetix^{®} 300 completely repermeabilized two different target arteries in two different animals 8 minutes after product administration in all arterial networks evaluated.

### 2.4 Clinical evaluation in humans

### 2.4.1. Knee osteoarthritis

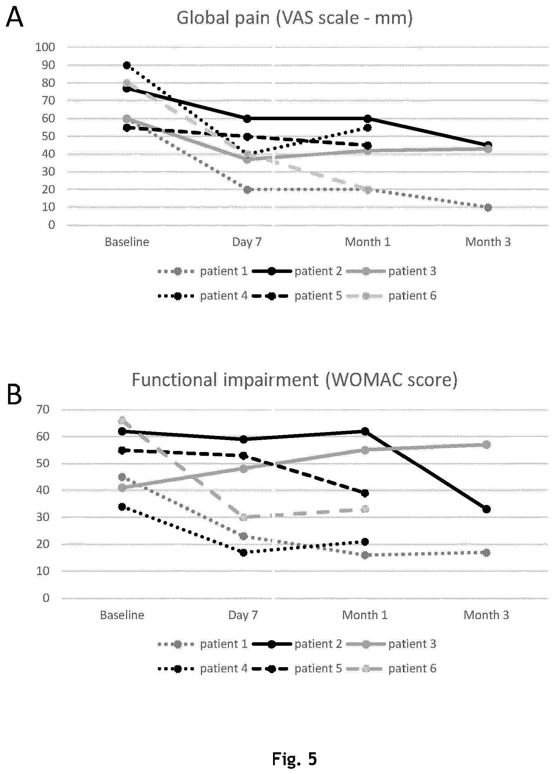

Clinical results at the latest available follow-up are provided in Figure 5, and summarized in the Table 4 below.

**Table 4. Efficacy of embolization as determined by pain and functional impairment assessment**

| | Last available follow-up | Pain | Functional impairment | Global result as reported by the patient |
|---|---|---|---|---|
| Patient 1 | 3 months | -83% | -62% | Clear improvement and resumption of physical activity |
| Patient 2 | 3 months | -42% | -47% | Clear improvement |
| Patient 3 | 3 months | -28% | 39% | No improvement |
| Patient 4 | 1 month | -39% | -38% | Clear improvement |
| Patient 5 | 1 month | -18% | -29% | Clear improvement and resumption of physical activity |
| Patient 6 | 1 month | -75% | -50% | Clear improvement |

6/6 patients showed a reduction in pain as reported on the VAS scale, with 5/6 patients reporting a reduction in functional impairment as determined on the WOMAC scale. When self-reporting, 5/6 patients furthermore reported a clear improvement, with 2/6 patients indicating that physical activity could be resumed.

During the course of embolization, patient 1 developed an erythema for 2 days and periarticular edema for 4 days, both of which were of low intensity and did not require the initiation of treatment. Patient 2 developed a mild erythema that did not require treatment, and which resolved within 4 hours. Patient management was slightly modified by placing an ice pack on the knee during embolization. With this modification, no erythema or edema has since been described.

None of the 6 patients developed any other sign of complication/toxicity.

### 2.4.2. Frozen shoulder

At 6 months post-embolization, clinical examination of the patient presenting a frozen shoulder showed a mild improvement in shoulder mobility with 20° gained in external rotation, and a very marked improvement in pain, with more than a 50% reduction in analgesic treatment with opiates.

### 3. Conclusions

In their study using an osteoarthrosis swine model, the inventors have surprisingly shown the efficacy of the emulsion according to the invention in treating inflammatory hypervascularization associated with a musculoskeletal disorder. In particular, the emulsion successfully embolized hypervascularized areas, as was confirmed by the immediate disappearance of hypervascularization in all embolized joints. Furthermore, hypervascularization was successfully treated regardless of whether a selective or non-selective approach was used for embolization. Importantly, the procedure was safe with patency of the donor artery at angiographic control observed in all joints. Non-target embolization was not observed in the selective approach. While transient skin ischemia was observed during the perioperative period when non-selective embolization was performed, this disappeared a few days after the procedure without any histological evidence at 14 days and no clinical signs of pain/discomfort.

The inventors have also shown that transitory embolization induced by the emulsion of the invention is reproducible and thus predictable.

A clinical trial in humans has further confirmed the efficacy of the emulsion according to the invention in treating inflammatory hypervascularization. Indeed, following embolization with the emulsion, 5/6 patients with knee osteoarthritis reported reduced pain and improved joint functionality at the latest available follow-up, with only 2/6 patients reporting any minor side effects. The surprising effect of the emulsion provided herein was further confirmed in an additional patient with frozen shoulder.

### References

Alfredson H, Ohberg L, Forsgren S, Is vasculo-neural ingrowth the cause of pain in chronic Achilles tendinosis? An investigation using ultrasonography and colour Doppler, immunohistochemistry, and diagnostic injections. Knee Surg Sports Traumatol Arthrosc. 2003, 11 (5): 334-338.
Bagla S, Piechowiak R, Hartman T, Orlando J, Del Gaizo D, Isaacson A. Genicular Artery Embolization for the Treatment of Knee Pain Secondary to Osteoarthritis. J Vasc Interv Radiol. 2020 Jul;31(7):1096-1102.
Bonnet CS, Walsh DA, Osteoarthritis, angiogenesis and inflammation. Rheumatology, 2005; 44(1): 7-16.
Isaacson and Bagla, Randomized Placebo-Controlled Single Blinded Study of Geniculate Artery Embolization for Knee Pain Secondary to Osteoarthritis, NCT number: NCT03362957, 2018.
James SL, Abate D, Abate KH, et al. Global, regional, and national incidence, prevalence, and years lived with disability for 354 diseases and injuries for 195 countries and territories, 1990-2017: a systematic analysis for the Global Burden of Disease Study 2017. Lancet 2018; 392: 1789-858.
Klamroth R, Gottstein S, Essers E, Landgraf H, Wilaschek M, Oldenburg J. Successful angiographic embolization of recurrent elbow and knee joint bleeds in seven patients with severe haemophilia. Haemophilia 2009; 15:247-252
McConnell S, Kolopack P, Davis AM. The Western Ontario and McMaster Universities Osteoarthritis Index (WOMAC): a review of its utility and measurement properties. Arthritis Rheum. 2001 Oct;45(5):453-61
Okuno Y, Matsumura N, Oguro S. Transcatheter arterial embolization using imipenem/cilastatin sodium for tendinopathy and enthesopathy refractory to nonsurgical management. J Vasc Interv Radiol 2013; 24:787-792
Shaffer, B; Tibone, JE, Kerlan, RK. Frozen shoulder. A long-term follow-up. J Bone Joint Surg Am 1992; 74(5): 738-746
United States Bone and Joint Initiative: The Hidden Impact of Musculoskeletal Disorders on Americans, Executive Summary, 4th ed., 2018. Rosemont, IL. Available at http://www.boneandjointburden.org. Accessed on Oct. 5, 2020.
Wolff, J. Physiology and Pharmacology of Iodized Oil in Goiter Prophylaxis. Medicine 2001; 80(1): 20-36.

## Claims

1. Embolizing emulsion comprising iodinated oil and an aqueous phase comprising a water-soluble contrast agent for use in the treatment of inflammatory hypervascularization associated with a musculoskeletal disorder.

2. Embolizing emulsion for use according to claim 1, wherein the iodinated oil comprises a mixture of ethyl esters of iodinated and non-iodinated fatty acids of poppyseed oil.

3. Embolizing emulsion for use according to claim 1 or 2, wherein the water-soluble contrast agent is an iodinated contrast agent.

4. Embolizing emulsion for use according to claim 3, wherein said water-soluble iodinated contrast agent is selected from the group consisting of ioversol, iopamidol, iomeprol, iopromide, iohexol, iobitridol, and iodixanol, preferably ioversol.

5. Embolizing emulsion for use according to any one of claims 1 to 4, wherein said emulsion is a water-in-oil emulsion.

6. Embolizing water-in-oil emulsion for use according to claim 5, wherein the ratio of iodinated oil to the aqueous phase is at least 2:1 v/v.

7. Embolizing emulsion for use according to any one of claims 3 to 6, wherein the concentration of iodine in the aqueous phase is comprised between 240 and 400 mg/mL.

8. Embolizing emulsion for use according to any one of claims 1 to 7, wherein the viscosity of the composition is comprised between 40 et 140 mPa.s at 37°C.

9. Embolizing emulsion for use according to any one of claims 1 to 8, wherein the emulsion droplet size is comprised between 10 and 100 µm.

10. Embolizing emulsion for use according to any one of claims 1 to 9, wherein said composition:
a) does not comprise a chemotherapeutic anticancer agent, and/or
b) does not comprise nanoparticles or polymeric particles, preferably wherein said composition does not comprise any embolic particle.

11. Embolizing emulsion for use according to any one of claims 1 to 10, wherein the musculoskeletal disorder affects the elbow, knee, wrist, shoulder, hip, heel, ankle, thumb and/or spine.

12. Embolizing emulsion for use according to any one of claims 1 to 10, wherein the musculoskeletal disorder is selected from enthesopathies, tendinopathies, inflammatory rheumatoid diseases and carpel tunnel syndrome.

13. Embolizing emulsion for use according to claim 12, wherein:
- the enthesopathy is selected from Achilles tendon enthesopathy, epicondylitis, ankylosing spondylitis, Plantar fasciitis, shoulder rotator cuff syndrome, enthesopathy of elbow region, enthesopathy of wrist and/or carpus, olecranon bursitis, prepatellar bursitis, hand or wrist bursitis, enthesopathy of hip region, hip bursitis, enthesopathy of knee, enthesopathy of ankle, enthesopathy of tarsus, and enthesopathy of calcaneus,
- the tendinopathy is selected from shoulder tendinitis, calcific tendinitis, Achilles tendinitis, biceps tendinitis, quadriceps tendinosis, lateral epicondylitis, medial epicondylitis, De Quervain's tenosynovitis, stenosing tenosynovitis, wrist tenosynovitis, patellar tendinopathy, or
- the inflammatory rheumatoid disease is selected from osteoarthritis, cervical spondylosis, rheumatoid arthritis (RA), acute crystal arthritis, spondyloarthropathies, psoriatic arthritis), Sjogren's syndrome, scleroderma, infectious arthritis, plantar fasciosis, juvenile idiopathic arthritis, polymyalgia rheumatica, fibromyalgia, lupus, vasculitis.

14. Embolizing emulsion for use according to any one of claims 1 to 13, wherein the musculoskeletal disorder is resistant or refractory to treatment with oral analgesics, anti-inflammatory medication, physical therapy, and/or corticosteroid injections.

15. Embolizing emulsion for use according to any one of claims 1 to 14, wherein said treatment comprises administering the embolizing emulsion to at least one target artery supplying blood to the affected tissue(s).

## Patentansprüche

1. Embolisierende Emulsion, die jodiertes Öl und eine wässrige Phase umfasst, die ein wasserlösliches Kontrastmittel umfasst, zur Verwendung bei der Behandlung von entzündlicher Hypervaskularisation in Verbindung mit einer Muskel-Skelett-Erkrankung.

2. Embolisierende Emulsion zur Verwendung nach Anspruch 1, wobei das jodierte Öl ein Gemisch aus Ethylestern von jodierten und nicht jodierten Fettsäuren von Mohnöl umfasst.

3. Embolisierende Emulsion zur Verwendung nach Anspruch 1 oder 2, wobei das wasserlösliche Kontrastmittel ein jodiertes Kontrastmittel ist.

4. Embolisierende Emulsion zur Verwendung nach Anspruch 3, wobei das wasserlösliche jodierte Kontrastmittel ausgewählt ist aus der Gruppe, die besteht aus Ioversol, Iopamidol, Iomeprol, Iopromid, Iohexol, Iobitridol und Iodixanol, vorzugsweise Ioversol.

5. Embolisierende Emulsion zur Verwendung nach einem der Ansprüche 1 bis 4, wobei die Emulsion eine Wasser-in-Öl-Emulsion ist.

6. Embolisierende Wasser-in-Öl-Emulsion zur Verwendung nach Anspruch 5, wobei das Verhältnis von jodiertem Öl zu der wässrigen Phase mindestens 2:1 v/v beträgt.

7. Embolisierende Emulsion zur Verwendung nach einem der Ansprüche 3 bis 6, wobei die Konzentration von Jod in der wässrigen Phase zwischen 240 und 400 mg/ml beträgt.

8. Embolisierende Emulsion zur Verwendung nach einem der Ansprüche 1 bis 7, wobei die Viskosität der Zusammensetzung bei 37°C zwischen 40 und 140 mPa.s beträgt.

9. Embolisierende Emulsion zur Verwendung nach einem der Ansprüche 1 bis 8, wobei die Emulsionströpfchengröße zwischen 10 und 100 µm beträgt.

10. Embolisierende Emulsion zur Verwendung nach einem der Ansprüche 1 bis 9, wobei die Zusammensetzung:
a) kein chemotherapeutisches Krebsmedikament umfasst, und/oder
b) keine Nanopartikel oder Polymerpartikel umfasst, wobei die Zusammensetzung vorzugsweise keine Embolisationspartikel umfasst.

11. Embolisierende Emulsion zur Verwendung nach einem der Ansprüche 1 bis 10, wobei die Muskel-Skelett-Erkrankung den Ellenbogen, das Knie, das Handgelenk, die Schulter, die Hüfte, die Ferse, den Knöchel, den Daumen und/oder die Wirbelsäule beeinträchtigt.

12. Embolisierende Emulsion zur Verwendung nach einem der Ansprüche 1 bis 10, wobei die Muskel-Skelett-Erkrankung ausgewählt ist aus Enthesopathien, Tendinopathien, entzündlichen rheumatischen Erkrankungen und Karpaltunnelsyndrom.

13. Embolisierende Emulsion zur Verwendung nach Anspruch 12, wobei:
- die Enthesopathie ausgewählt ist aus Achillessehnen-Enthesopathie, Epicondylitis, ankylosierender Spondylitis, Plantarfasziitis, Rotatorenmanschetten-Syndrom der Schulter, Enthesopathie der Ellenbogenregion, Enthesopathie des Handgelenks und/oder des Handwurzelknochens, Olekranonbursitis, präpatellarer Bursitis, Hand- oder Handgelenksbursitis, Enthesopathie der Hüftregion, Hüftbursitis, Enthesopathie des Knies, Enthesopathie des Knöchels, Enthesopathie des Fußwurzelknochens und Enthesopathie des Fersenbeins;
- die Tendinopathie ausgewählt ist aus Schultersehnenentzündung, kalzifizierender Sehnenentzündung, Achillessehnenentzündung, Bizeps-Sehnenentzündung, Quadrizeps-Sehnenentzündung, lateraler Epicondylitis, medialer Epicondylitis, De-Quervain-Sehnenscheidenentzündung, stenosierender Sehnenscheidenentzündung, Handgelenk-Sehnenscheidenentzündung, Patellasehnenentzündung, oder
- die entzündliche rheumatische Erkrankung ausgewählt ist aus Osteoarthritis, zervikaler Spondylose, rheumatoider Arthritis (RA), akuter Kristallarthritis, Spondyloarthropathien, Psoriasis-Arthritis, Sjögren-Syndrom, Sklerodermie, infektiöser Arthritis, Plantarfasziose, juveniler idiopathischer Arthritis, Polymyalgia rheumatica, Fibromyalgie, Lupus, Vaskulitis.

14. Embolisierende Emulsion zur Verwendung nach einem der Ansprüche 1 bis 13, wobei die Muskel-Skelett-Erkrankung resistent oder behandlungsrefraktär gegenüber einer Behandlung mit oralen Schmerzmitteln, entzündungshemmenden Medikamenten, Physiotherapie und/oder Kortikosteroid-Injektionen ist.

15. Embolisierende Emulsion zur Verwendung nach einem der Ansprüche 1 bis 14, wobei die Behandlung das Verabreichen der embolisierenden Emulsion in mindestens eine Zielarterie umfasst, welche das/die betroffene/n Gewebe mit Blut versorgt bzw. versorgen.

## Revendications

1. Émulsion embolisante comprenant une huile iodée et une phase aqueuse comprenant un agent de contraste hydrosoluble, pour utilisation dans le traitement de l'hypervascularisation inflammatoire associée à un trouble musculo-squelettique.

2. Émulsion embolisante pour son utilisation selon la revendication 1, dans laquelle l'huile iodée comprend un mélange d'esters éthyliques d'acides gras iodés et non iodés d'huile de pavot.

3. Émulsion embolisante pour son utilisation selon la revendication 1 ou 2, dans laquelle l'agent de contraste hydrosoluble est un agent de contraste iodé.

4. Émulsion embolisante pour son utilisation selon la revendication 3, dans laquelle ledit agent de contraste iodé hydrosoluble est choisi parmi le groupe constitué de l'ioversol, de l'iopamidol, de l'iomeprol, de l'iopromide, de l'iohexol, de l'iobitridol et de l'iodixanol, de préférence l'ioversol.

5. Émulsion embolisante pour son utilisation selon l'une quelconque des revendications 1 à 4, dans laquelle ladite émulsion est une émulsion eau-dans-huile.

6. Émulsion embolisante eau-dans-huile pour son utilisation selon la revendication 5, dans laquelle le rapport entre l'huile iodée et la phase aqueuse est d'au moins 2:1 en volume.

7. Émulsion embolisante pour son utilisation selon l'une quelconque des revendications 3 à 6, dans laquelle la concentration en iode dans la phase aqueuse est comprise entre 240 et 400 mg/mL.

8. Émulsion embolisante pour son utilisation selon l'une quelconque des revendications 1 à 7, dans laquelle la viscosité de la composition est comprise entre 40 et 140 mPa.s à 37 °C.

9. Émulsion embolisante pour son utilisation selon l'une quelconque des revendications 1 à 8, dans laquelle la taille des gouttelettes de l'émulsion est comprise entre 10 et 100 µm.

10. Émulsion embolisante pour son utilisation selon l'une quelconque des revendications 1 à 9, dans laquelle ladite composition :
a) ne comprend pas d'agent chimiothérapeutique anticancéreux, et/ou
b) ne comprend pas de nanoparticules ou de particules polymères, de préférence ladite composition ne comprenant aucune particule embolique.

11. Émulsion embolisante pour son utilisation selon l'une quelconque des revendications 1 à 10, dans laquelle le trouble musculo-squelettique affecte le coude, le genou, le poignet, l'épaule, la hanche, le talon, la cheville, le pouce et/ou la colonne vertébrale.

12. Émulsion embolisante pour son utilisation selon l'une quelconque des revendications 1 à 10, dans laquelle le trouble musculo-squelettique est choisi parmi les enthésopathies, les tendinopathies, les maladies rhumatoïdes inflammatoires et le syndrome du canal carpien.

13. Émulsion embolisante pour son utilisation selon la revendication 12, dans laquelle :
- l'enthésopathie est choisie parmi l'enthésopathie du tendon d'Achille, l'épicondylite, la spondylarthrite ankylosante, la fasciite plantaire, le syndrome de la coiffe des rotateurs de l'épaule, l'enthésopathie de la région du coude, l'enthésopathie du poignet et/ou du carpe, la bursite olécrânienne, la bursite prépatellaire, la bursite de la main ou du poignet, l'enthésopathie de la région de la hanche, la bursite de la hanche, l'enthésopathie du genou, l'enthésopathie de la cheville, l'enthésopathie du tarse et l'enthésopathie du calcanéum,
- la tendinopathie est choisie parmi la tendinite de l'épaule, la tendinite calcifiante, la tendinite d'Achille, la tendinite du biceps, la tendinose du quadriceps, l'épicondylite latérale, l'épicondylite médiale, la ténosynovite de De Quervain, la ténosynovite sténosante, la ténosynovite du poignet, la tendinopathie rotulienne, ou
- la maladie rhumatoïde inflammatoire est choisie parmi l'arthrose, la spondylose cervicale, la polyarthrite rhumatoïde (PR), l'arthrite cristalline aiguë, les spondyloarthropathies, l'arthrite psoriasique, le syndrome de Sjögren, la sclérodermie, l'arthrite infectieuse, la fasciose plantaire, l'arthrite juvénile idiopathique, la polymyalgie rhumatismale, la fibromyalgie, le lupus et la vascularite.

14. Émulsion embolisante pour son utilisation selon l'une quelconque des revendications 1 à 13, dans laquelle le trouble musculo-squelettique est résistant ou réfractaire à un traitement par analgésiques oraux, médicaments anti-inflammatoires, kinésithérapie et/ou injections de corticostéroïdes.

15. Émulsion embolisante pour son utilisation selon l'une quelconque des revendications 1 à 14, dans laquelle ledit traitement comprend l'administration de l'émulsion embolisante dans au moins une artère cible irriguant le ou les tissus affecté(s).
